Europäisches Patentamt

European Patent Office

Office européen des brevets

(11) Veröffentlichungsnummer: **0 173 130**
**B1**

(12) # EUROPÄISCHE PATENTSCHRIFT

(45) Veröffentlichungstag der Patentschrift:
**07.12.88**

(21) Anmeldenummer: **85109885.5**

(22) Anmeldetag: **06.08.85**

(51) Int. Cl.⁴: **G 01 N 24/02**, G 01 N 24/08

(54) **Einrichtung für die Kernspin-Tomographie.**

(30) Priorität: **20.08.84 DE 3430625**

(43) Veröffentlichungstag der Anmeldung:
**05.03.86 Patentblatt 86/10**

(45) Bekanntmachung des Hinweises auf die Patenterteilung:
**07.12.88 Patentblatt 88/49**

(84) Benannte Vertragsstaaten:
**DE FR GB NL**

(56) Entgegenhaltungen:
**EP-A- 0 098 426**
**EP-A- 0 105 550**
**GB-A- 2 126 731**
**US-A- 3 564 398**

(73) Patentinhaber: **Siemens Aktiengesellschaft Berlin und München, Wittelsbacherplatz 2, D-8000 München 2 (DE)**

(72) Erfinder: **Weigert, Kurt, Grillparzerstrasse 26, D-8510 Fürth (DE)**

## Beschreibung

Die Erfindung betrifft eine Einrichtung für die Kernspin-Tomographie zur bildlichen Darstellung von Teilbereichen aus dem Innern eines Untersuchungsobjektes mit Spulen zum Anlegen von magnetischen Grund- und Gradientenfeldern an das Untersuchungsobjekt und einer Antenne zum Erfassen der Auslenkung der Kernspins des Untersuchungsobjektes aus ihrer Gleichgewichtslage durch hochfrequente magnetische Anregungsimpulse, wobei Teile des Kernspin-Tomographiegerätes in einer abgeschirmten Kabine untergebracht sind.

Ein derartiges Gerät ist aus der EP-A 0 105 550 bekannt. Dabei ist der Untersuchungsraum des Kernspin-Tomographiegerätes mit einem Faraday-Käfig umgeben, um den Untersuchungsraum gegen elektromagnetische Streufelder abzuschirmen und den Q-Faktor der Sende-/Empfangsspulenanordnung zu verbessern. Um zu verhindern, dass die den Faraday-Schirm durchdringenden Leiter als Antennen wirken, ist jeder derartige Leiter mit einem Filter versehen. Solche Filter sind jedoch aufwendig. Um daher die Zahl der den Faraday-Schirm durchdringenden Leitungen gering zu halten, umfasst dieser bevorzugt nur den eigentlichen Untersuchungsraum mit Sende- und Empfangsspulen.

Aus der GB-A 2 126 731 ist ein Kernspin-Tomographiegerät bekannt, bei dem die nach der Auslenkung der Kernspins entstehenden Messsignale über einen Lichtwellenleiter einer Verarbeitungselektronik zugeführt werden. Bei der dargestellten Anordnung sind keine Abschirmungen vorgesehen.

Bei Kernspin-Tomographiegeräten tritt das Problem auf, dass zur Erfassung bewegter Körperbereiche eine Triggerung des Messvorganges in Abhängigkeit von der Atmung und/oder vom EKG des Patienten erfolgen muss. Hierzu kann ein Atemdetektor und können EKG-Elektroden vorgesehen werden, die von der Atmung und der Herztätigkeit abhängige elektrische Signale erzeugen, die die Triggerung des Messvorganges bewirken.

Der Erfindung liegt die Aufgabe zugrunde, ein Gerät der eingangs genannten Art so auszubilden, dass störungsfrei physiologische Messsignale, die insbesondere von der Atmung und vom EKG abhängen, gebildet werden, die eine einwandfreie Triggerung des Messvorganges ermöglichen.

Diese Aufgabe wird erfindungsgemäss dadurch gelöst, dass zur Bildung von physiologischen Messsignalen in der abgeschirmten Kabine eine Messelektronik mit einem elektrisch-optischen Wandler angeordnet ist, von dem ein Lichtwellenleiter zu einer ausserhalb der Kabine vorgesehenen Verarbeitungselektronik mit einem opto-elektrischen Wandler führt, und dass dem elektrisch-optischen Wandler ein Slew-Rate-Filter vorgeschaltet ist.

Bei dem erfindungsgemässen Kernspin-Tomographiegerät erfolgt eine optische Weiterleitung der von der Messelektronik in geeigneter Weise aufbereiteten physiologischen Messsignale, so dass sich insbesondere das Schalten der Gradienten nicht störend auf diese Messsignale auswirkt. Die den Messsignalen überlagerten, von den geschalteten Gradienten herrührenden Störsignale werden durch ein Slew-Rate-Filter ausgeblendet.

Eine besonders störungsfreie und selbst keine Störungen verursachende Signalübertragung ergibt sich, wenn der elektrisch-optische Wandler eine analoge Lichtmodulation bewirkt.

Einzelheiten der Erfindung ergeben sich aus der nachfolgenden Beschreibung eines Ausführungsbeispieles anhand der Zeichnung in Verbindung mit den Unteransprüchen. Es zeigen:

Fig. 1 ein Kernspin-Tomographiegerät nach der Erfindung,

Fig. 2 eine Einzelheit des Gerätes gemäss Fig. 1 und

Fig. 3 bis 5 Kurven zur Erläuterung des Gerätes gemäss den Kurven 1 und 2.

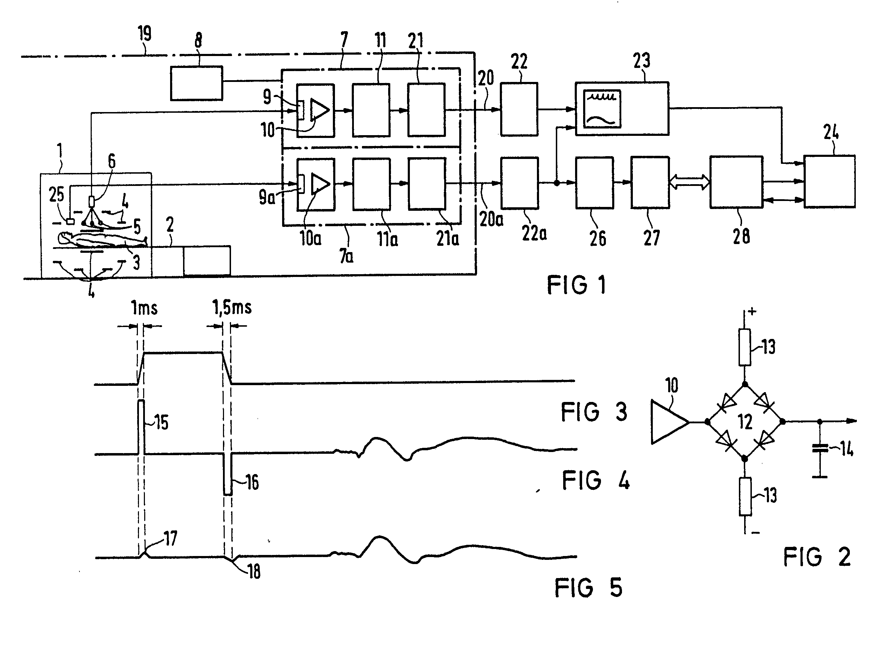

In der Figur 1 ist ein Kernspin-Tomographiegerät 1 dargestellt, das eine Patientenliege 2 aufweist, auf der ein Patient 3 liegt. Zur Untersuchung des Patienten 3 weist das Kernspin-Tomographiegerät 1 eine schematisch dargestellte Spulenanordnung 4 zum Anlegen von magnetischen Grund- und Gradientenfeldern an das Untersuchungsobjekt auf, die eine Antenne zum Erfassen der Auslenkung der Kernspins des Untersuchungsobjektes aus ihrer Gleichgewichtslage durch hochfrequente magnetische Anregungsimpulse aufweist. Die Messsignale werden in bekannter Weise zu einem Bild einer Schicht des Patienten 3 weiterverarbeitet.

Zur Triggerung der Messwerterzeugung in Abhängigkeit vom EKG sind dem Patienten 3 z.B. EKG-Elektroden 5 zugeordnet, die über Widerstände 6 in den Elektrodenleitungen an einem EKG-Verstärker 7 angeschlossen sind. Der EKG-Verstärker 7 wird von einem Netzteil 8 versorgt und weist an seinem Eingang ein Hochfrequenzfilter 9 und eine nachgeschaltete breitbandige Verstärkerstufe 10 mit einem Durchlassfrequenzbereich zwischen beispielsweise 0,1 und 10 kHz auf. Die Verstärkung der Verstärkerstufe 10 ist so gewählt, dass die durch die Gradientenimpulse erzeugten Störimpulse noch innerhalb des linearen Arbeitsbereiches der Verstärkerstufe 10 liegen, d.h. die Verstärkerstufe dadurch nicht übersteuert wird. Am Ausgang der Verstärkerstufe 10 ist ein Slew-Rate-Filter 11 angeordnet. Durch das Slew-Rate-Filter 11 werden die beim Anstieg und Abfall der Gradientenimpulse (Fig. 3) entstehenden Störimpulse wirksam unterdrückt.

Der Aufbau des Slew-Rate-Filters 11 ist in der Figur 2 genauer dargestellt. Die Figur 2 zeigt, dass das Slew-Rate-Filter 11 eine Gleichrichterbrücke 12 aufweist, die über zwei gleich grosse Widerstände 13 am Plus- und Minus-Pol einer Gleichspannungsquelle liegt. Am Ausgang des Slew-Rate-Filters 11 liegt ein Kondensator 14.

Die Gradientenimpulse gemäss Figur 3 haben eine Anstiegs- und eine Abfallzeit von 1 ms bzw. 1,5 ms. Während dieses Anstieges bzw. Abfalles weist das Signal vor dem Slew-Rate-Filter 11 gemäss Figur 4 Störimpulse 15, 16 auf. Nach dem Slew-Rate-Filter 11 bewirken diese Störimpulse 15, 16 gemäss Figur 5 Reststörimpulse 17, 18, deren Amplitude nur noch von der Impulsbreite (Fig. 4), nicht jedoch von der Amplitude der Impulse gemäss Figur 4 abhängt. Das Slew-Rate-Filter 11 unterdrückt demgemäss die beim Anstieg und Abfall der Gradientenimpulse entstehenden Störimpulse wirksam.

Die Weiterleitung des Ausgangssignales des Slew-Rate-Filters 11 aus der das Kernspin-Tomographiegerät 1 aufnehmenden, abgeschirmten Kabine 19 erfolgt über einen Lichtwellenleiter 20, dem ein elektrisch-optischer Wandler 21 vorgeschaltet ist. Die Übertragung erfolgt dabei durch analoge Modulation des Lichtes, so dass Störungen weiterhin reduziert werden. Ausserhalb der Kabine 19 ist ein opto-elektrischer Wandler 22 vorgesehen, der einem Patientenmonitor 23 das EKG-Signal zuführt. Durch den Patientenmonitor 23 kann über eine Messsequenzsteuerung 24 eine Triggerung des Messvorganges in Abhängigkeit vom EKG erfolgen.

Um die Bildbeeinflussung durch die Elektroden sowie die Weiterleitung der Hochfrequenz über die Elektrodenleitungen bzw. den durch die Hochfrequenz in die Elektrodenleitungen induzierten Strom möglichst klein zu halten, sind die Längswiderstände 6 in den Elektrodenleitungen entsprechend dimensioniert.

Eine analoge Signalübertragung erfolgt für das von einem Atemdetektor 25 erzeugte Atemsignal. Die den Komponenten 9, 10, 11, 20, 21 und 22 entsprechenden Komponenten sind dabei mit 9a, 10a, 11a, 20a, 21a und 22a bezeichnet. Der optoelektrische Wandler 22a bewirkt eine Wiedergabe der Atemkurve auf dem Patientenmonitor 23 und steuert die Messsequenzsteuerung 24 über ein 50/60-Hz-Filter 26, einen Analog-Digital-Konverter 27 und einen Mikrocomputer 28.

## Patentansprüche

1. Einrichtung für ein Kernspin-Tomographiegerät zur bildlichen Darstellung von Teilbereichen aus dem Innern eines Untersuchungsobjektes (3) mit Spulen (4) zum Anlegen von magnetischen Grund- und Gradientenfeldern an das Untersuchungsobjekt (3) und einer Antenne zum Erfassen der Auslenkung der Kernspins des Untersuchungsobjektes (3) aus ihrer Gleichgewichtslage durch hochfrequente magnetische Anregungsimpulse, wobei Teile des Kernspin-Tomographiegerätes (1) in einer abgeschirmten Kabine (19) untergebracht sind, dadurch gekennzeichnet, dass zur Bildung von physiologischen Messsignalen in der abgeschirmten Kabine (19) eine Messelektronik (7, 7a) mit einem elektrisch-optischen Wandler (21, 21a) angeordnet ist, von dem ein Lichtwellenleiter (20, 20a) zu einer ausserhalb der Kabine (19) vorgesehenen Verarbeitungselektronik (22, 22a, 23, 24, 26, 27, 28) mit einem opto-elektrischen Wandler (22, 22a) führt, und dass dem elektrisch-optischen Wandler (21, 21a) ein Slew-Rate-Filter (11, 11a) vorgeschaltet ist.

2. Einrichtung nach Anspruch 1, dadurch gekennzeichnet, dass der elektrisch-optische Wandler (21, 21a) eine analoge Lichtmodulation bewirkt.

3. Einrichtung nach Anspruch 1 oder 2, dadurch gekennzeichnet, dass die Messelektronik (7, 7a) am Eingang ein Hochfrequenzfilter (9, 9a) und einen nachgeschalteten breitbandigen Verstärker (10, 10a) aufweist.

## Claims

1. Arrangement for nuclear spin tomography equipment for the pictorial representation of partial areas from the inside of an examination subject (3) having coils (4) for the application of magnetic basic and gradient fields to the examination subject (3) and having an antenna for detecting the deflection of the nuclear spin of the examination subject (3) from his/her position of equilibrium through high-frequency magnetic stimulation pulses, parts of the nuclear spin tomography equipment (1) being accomodated in a screened cabin (19), characterised in that for the purpose of establishing physiological measuring signals in the screened cabin (19) there is arranged a measuring electronics unit (7, 7a) with an electrical-optical transducer (21, 21a) from which there leads a light waveguide (20, 20a) to a processing electronics unit (22, 22a, 23, 24, 26, 27, 28), provided outside the cabin (19), with an opto-electric transducer (22, 22a), and in that connected before the electrical-optical transducer (21, 21a) there is a slew-rate-filter (11, 11a).

2. Arrangement according to claim 1, characterised in that the electrical-optical transducer (21, 21a) effects an analog light modulation.

3. Arrangement according to claim 1 or 2, characterised in that the measuring electronics unit (7, 7a) has at the input a high-frequency filter (9, 9a) and a subsequently-connected wide-band amplifier (10, 10a).

## Revendication

1. Dispositif pour un appareil de tomographie à spin nucléaire servant à réaliser la représentation imagée de zones partielles de l'intérieur d'un objet d'examen (3), comportant des bobines (4) servant à appliquer un champ magnétique de base et des champs magnétiques irrotationnels à un object d'examen (3) et une antenne pour détecter la déviation du spin nucléaire de l'objet d'examen (3) à partir de sa position d'équilibre sous l'effet d'impulsions d'excitation magnétiques à haute fréquence, des parties de l'appareil (1) de tomographie à spin nucléaire étant logées dans une cabine blindée (19), caractérisé par le fait que pour la formation de signaux de mesure physiologiques, dans la cabine blindée (19) se trouve disposé un système électronique de mesure (7, 7a), qui comporte un transducteur électro-optique (21, 21a), d'où part un guide d'ondes optiques (20, 20a) aboutissant à un système électronique de traitement (22, 22a, 23, 24, 26, 27, 28) prévu à l'extérieur de la cabine (19) et comportant un transducteur électro-optique (22, 22a), et qu'un filtre Slew-Rate (11, 11a) est branché en amont du transducteur électro-optique (21, 21a).

2. Dispositif suivant la revendication 1, caractérisé par le fait que le transducteur électro-optique (21, 21a) réalise une modulation analogique de la lumière.

3. Dispositif suivant la revendication 1 ou 2, caractérisé par le fait que le système électronique de mesure (7, 7a) comporte, à son entrée, un filtre (9, 9a) de suppression des hautes fréquences et un amplificateur à large bande (10, 10a) branché en aval.

EP 0 173 130 B1

FIG 1

1ms    1,5ms

FIG 3

FIG 4

FIG 5

FIG 2